# EUROPÄISCHE PATENTSCHRIFT

(11) **EP 0 989 098 B1**
(45) Veröffentlichungstag und Bekanntmachung des Hinweises auf die Patenterteilung: **09.04.2003**
(21) Anmeldenummer: 99250333.4
(22) Anmeldetag: 21.09.1999
(51) Int. Cl.: C02F 3/34, B09C 1/10, A62D 3/00, C12N 1/20

(54) **Bakterienstamm Comamonas testosteroni MC1 und Verfahren zur mikrobiellen Dekontamination von mit Phenoxy-Herbiziden belasteten Materialien**
Bacterial strain Comamonas Testosteroni MC1 and process for the microbial decontamination of materials polluted with phenoxyacetic acid herbicides
Souche bactérienne comamonas testosteroni mc1 et procédé de décontamination microbienne de matériaux pollués par des herbicides à base d'acide phenoxyacétique

(30) Priorität: 21.09.1998 DE 19845784
(43) Veröffentlichungstag der Anmeldung: 29.03.2000
(73) Patentinhaber: UFZ-UMWELTFORSCHUNGSZENTRUM Leipzig-Halle GmbH, 04318 Leipzig (DE)
(72) Erfinder: Müller, Roland H., Dr., 04347 Leipzig (DE); Babel, Wolfgang, Prof. Dr., 04347 Leipzig (DE)
(74) Vertreter: Ziebig, Marlene, Dr. Dipl.-Chem.

(56) Entgegenhaltungen:
- DE-A- 19 730 653
- SMITH, A. ET AL.: "Degradation of waste 2,4-D residues using a soil bacteriium in a sprayer tank system" CAN. J. SOIL SCI., Bd. 71, Nr. 2, 1991, Seiten 243-246, XP000870297 Canada, Regina
- BOKHAMY M ET AL: "survival and activity of comamonas testosteroni in mixed population " WATER RESEARCH,NL,ELSEVIER SCIENCE PUBLISHERS, AMSTERDAM, Bd. 31, Nr. 11, 1. November 1997 (1997-11-01), Seiten 2802-2810, XP004094226 ISSN: 0043-1354

## Beschreibung

Die Erfindung betrifft den neuen Bakterienstamm *Comamonas testosteroni* MC1 sowie ein Verfahren zur mikrobiellen Dekontamination von Materialien, die mit einzelnen Herbiziden oder Gemischen von Herbiziden der Phenoxyalkansäure-Produktion belastet sind, wie z.B. mit 2, 4-Dichlorphenoxybuttersäure (2,4-DB), 4-Chlor-2-methylphenoxybuttersäure (MCPB), 2,4-Dichlorphenoxypropionsäure (2,4-DP), 4-Chlor-2-methylphenoxypropionsäure (MCPP), 2,4-Dichlorphenoxyessigsäure (2,4-D), 4-Chlor-2-methylphenoxyessigsäure (MCPA), 2,4-Dichlorphenol (DCP) und/oder 4-Chlor-2-methylphenol (MCP) bzw. mit deren Salzen.

Produktionssstandorte der chemischen Industrie, so auch die der Herbizid-Produktion, in denen jahrelang produziert worden ist, sind mit den Ausgangs-, Zwischen- und Endprodukten kontaminiert. Das betrifft sowohl die Produktionsanlage selbst als auch das umgebende Gelände einschließlich Wässer und Grundwässer.

Diese o.g. Verbindungen der Phenoxyalkansäure-Produktion 2,4-D 2,4-DP, 2,4-DB, MCPA, MCPP und MCPB sowie DCP und MCP sind toxisch und haben z.T. cancerogene und teratogene Wirkung. Bei der Demontage von Anlagen zur Produktion solcher Verbindungen fällt u.a. Bauschutt an, in der Regel als geschreddertes Material, der kontaminiert ist. Dieses Material muß dekontaminiert werden, soll es weiter verwendet und wieder eingebaut werden. Das ist sowohl zum Schutz exponierter Personen als auch zum Schutz von Flora und Fauna unbedingt notwendig. Diese Vorgehensweise ist ökologisch und auch ökonomisch sinnvoll. Eine Deponierung dagegen löst nicht die Probleme, da dies wenig umweltfreundlich und somit perspektivisch nicht zu akzeptieren ist.

Es ist bekannt, daß Mikroorganismen in der Lage sind, chlorierte aromatische Verbindungen, darunter Phenoxy-Herbizide produktiv, d.h. durch Wachstum unter Bildung von Biomasse, CO₂, Wasser und Salzsäure abzubauen. Dafür sind eine Reihe von Beispielen unter Nutzung von Rein- und Mischkulturen beschrieben (Tiedje et al. 1969, J. Agr. Food Chem. 17, 1021; Lappin 1985, Appl. Env. Microbiol. 49, 429; Kilpi 1980, Microbiol. Ecol. 6, 261; Pieper et al. 1988, Arch. Microbiol. 150, 95; Haugland et al. 1990, Appl. Env. Microbiol. 56, 1357; Horvath et al. 1990, Appl. Microbiol. Biotechnol. 33, 213; Oh und Tuovinen 1990, J. Ind. Microbiol. 6, 275; Short et al. 1990, Can. J. Microbiol. 36, 822).

Phenoxyherbizidverwertende Spezies sind allerdings, wenn sie in axenischer Kultur überhaupt zur produktiven Mineralisation der Phenoxy-Herbizide in der Lage sind, bezüglich der Breite des Substratspektrums eingeschränkt. Das gilt z.B. für *Ralstonia eutropha* JMP 134 (Pieper et al. 1988, Arch. Microbiol. 150, 95) und andere neutrophile Stämme (siehe Sinton et al. 1986, Enzyme Microb. Technol. 8, 395), aber auch für die bekannten alkaliphilen Stämme *Comamonas acidovorans* P4a (DE 196 08 319 C1; Hoffmann et al. 1996; Acta Biotechnol. 16, 121) und *Corynebacterium* sp. K2-17 (DE 196 54 624.9 C1; Mertingk et al. 1998, J. Basic Microbiol. 38, 283), die Phenoxyacetat- bzw. -butyrat-Derivate utilisieren.

Im Gegensatz dazu ist der bekannte Stamm *Rhodoferax* sp. P230 metabolisch außerordentlich versatil. Das Spektrum der von ihm verwerteten Verbindungen umfaßt sowohl die dichlorierten als auch die 4-chlor-2-methylsubstituierten Phenoxyacetat- und Phenoxypropionat-Derivate sowie die entsprechend substituierten Phenole (Ehrig et al. 1997, Acta Biotechnol. 17, 351). Dieser Stamm eignet sich deshalb prinzipiell zum Abbau von Mischkontaminationen, wie sie häufig auftreten.

Er hat allerdings den Nachteil, daß er nicht prototroph wächst, sondern auf bioorganische/biogene Zusätze angewiesen ist. Damit ist nicht nur die Kultivierung zum Zwecke der Bereitung eines Inokulums zur Initiierung des Abbaus kontaminierter Materialien aufwendig, sondern auch der eigentliche produktive Abbau. Sogenannte Suppline wie z.B. Vitamine oder Aminosäuren müssen rein oder in komplexer Form zugeführt werden, wodurch der Einsatz des Stammes *Rhodoferax* sp. P230 erheblich eingeschränkt ist. Darüber hinaus ist die Wachstumsrate und damit letztlich auch die Substrat-(Herbizid-)Verwertungsrate für diesen Mikroorganismus insgesamt relativ klein.

Der Erfindung lag deshalb die Aufgabe zugrunde, weitere Mikroorganismen zu finden, die für den komplexen Abbau von Herbiziden der Phenoxyalkansäure-Produktion und für die Kultivierung keine Suppline benötigen sowie möglichst hohe Stoffumsätze (Wachstumsraten) realisieren. Außerdem lag der Erfindung die Aufgabe zugrunde, unter Einsatz dieser Mikroorganismen ein anwendungsfähiges Verfahren zur mikrobiellen Dekontamination von mit insbesondere Phenoxyherbizid-Gemischen kontaminierten Materialien und Wässern zu entwickeln.

Die Aufgabe konnte dadurch gelöst werden, daß ein neuer Bakterienstamm gefunden wurde, der zum Abbau sowohl von 2,4-DP, MCPP, 2,4-D, MCPA als auch von DCP und/oder MCP in der Lage ist. Die Herbizid-Verbindungen können einzeln oder in beliebiger Kombination und beliebiger Konzentration zueinander vorliegen. Ihre Verwertung durch den neuen Bakterienstamm erfolgt simultan.

Es handelt sich um den Bakterienstamm *Comamonas testosteroni* MC1. Er wurde aus herbizidkontaminiertem Bauschutt isoliert und am 24.08.1998 bei der Deutschen Sammlung von Mikroorganismen und Zellkulturen GmbH, Braunschweig, DE unter der Nummer DSM 12392 hinterlegt.

Der Stamm wächst im leicht sauren bis stark alkalischen pH-Bereich und ist überraschend in der Lage, entgegen der in aller Regel angetroffenen Spezialisierung sowohl die Phenoxyacetat- als auch die Phenoxypropionat-Derivate einschließlich der entsprechenden substituierten Phenole zu mineralisieren.

Die Vermehrung des Stammes *Comamonas testosteroni* MC1 erfolgt *ex situ* nach an sich bekannten Verfahren kontinuierlich, diskontinuierlich oder mittels anderer Fütterungsstrategien, wie z.B. unter Nutzung von Salzen der n-Alkansäuren (Acetat, Butyrat); Dikarbonsäuren (Succinat) oder anderen Kohlenstoff/Energiequellen.

Wachstum und Vermehrung auf den heterotrophen Substraten einschließlich der chlorierten Phenole und/oder Phenoxyalkansäuren bzw. die produktive Degradation der Schadstoffe erfordern überraschenderweise nur das Hinzufügen einer geeigneten N- bzw. P-Quelle zum Medium. Ein Zusatz von Supplinen in Form komplexer Substrate wie z.B. Hefeextrakt, ist nicht erforderlich.

Der Stamm *Comamonas testosteroni* MC 1 kann auch direkt allein auf einem Herbizid ausgewählt aus 2,4-DP, MCPP, 2,4-D, MCPA, DCP oder MCP oder einem Gemisch von diesen Herbiziden als Kohlenstoff- und Energiequelle kultiviert werden. Die Wachstumsrate auf Herbiziden kann durch Zusatz eines weiteren nicht chlororganischen heterotrophen Auxiliarsubstrates gesteigert werden. Dies kann z.B. das Salz einer organische Säure wie z.B. Acetat oder Succinat sein. Das Mischungsverhältnis Problemstoff(e) : Auxiliarsubstrat kann dabei in weiten Grenzen von 10:1 bis 1:10 (in g/g) variiert werden.

Das erfindungsgemäße Verfahren zum Abbau der Herbizid-Verbindungen 2,4-DP, MCPP, 2,4-D, MCPA, DCP und/oder MCP erfolgt im leicht sauren bis stark alkalischen pH-Bereich von 6 bis 11,5, bevorzugt bei pH-Werten von 7-10, wobei vorzugsweise Temperaturen von 4 bis 38 °C gewählt werden und die Herbizide auch gleichzeitig als Kohlenstoff- und Energiequelle für Wachstum und Vermehrung genutzt werden können.

Die Dekontamination der Herbizide bzw. Herbizidgemische durch Wachstum und Vermehrung erfolgt bevorzugt in wäßrigen Eluaten aus belasteten festen Materialen. Es ist auch möglich, die Materialien direkt nach Befeuchtung zu dekontaminieren.

Der erfindungsgemäße Bakterienstamm *Comamonas testosteroni* MC1 ist zum simultanen Abbau von 2,4-DP, 2,4-D, MCPP, MCPA, DCP und/oder MCP geeignet, die vollständig und bevorzugt direkt aus dem belasteten Material abgebaut werden können, wodurch an sich schon ein breites Spektrum von Verbindungen der Phenoxyalkansäure-Herbizid-Produktion entsorgt werden kann. Bei kontinuierlicher Vermehrung des Stammes werden gleichzeitig die belasteten Materialien, wie z.B. Böden, Mauerwerk oder Wässer kontinuierlich dekontaminiert.

Das erfindungsgemäße Verfahren wird vorzugsweise in Festbettreaktoren nach dem Tropfkörperprinzip, mittels Mietentechnik oder in Flüssigphasenreaktoren durchgeführt, wobei der Prozeß kontinuierlich, partiell kontinuierlich oder diskontinuierlich betrieben werden kann. Bei diskontinuierlicher Dekontamination wird den belasteten Wässern oder Materialien Biomasse der Mikroorganismenstämme in einer bevorzugten Konzentration von 0,01 bis 1 g/l bzw. /kg zugesetzt.

Unter den genannten Bedingungen ist der erfindungsgemäße Stamm *C. testosteroni* MC1 hervorragend geeignet, 2,4-DP/MCPP, 2,4-D/MCPA sowie DCP/MCP vollständig abzubauen. Dies prädestiniert ihn für die Schadstoffbeseitigung von mit solchen Substanzen und insbesondere solchen Substanzgemischen kontaminierten Materialien. Es wird eine Substratumsatzrate für ein Herbizidgemisch von mindestens 18 g/m³*h erreicht, wobei die Effizienz des Abbaus bei diesen hohen Raten überraschend noch über 90 % liegt.

Erfindungsgemäß wird in einer bevorzugten Ausführungsvariante dieser neue Stamm *Comamonas testosteroni* MC1 mit einem 2,4-DB/MCPB mineralisierenden Stamm kombiniert, wodurch das Spektrum des Schadstoffabbaus noch erweitert wird und der vollständige Abbau von Schadstoffen der Phenoxyalkansäure-Produktion erreicht wird.

Besonders bevorzugt wird als 2,4-DB/MCPB verwertender Stamm der Stamm *Corynebacterium sp. K2-17* (DSM 11288) eingesetzt. *Corynebacterium sp. K2-17* wird ebenfalls nach an sich üblichen Methoden kultiviert (DE 196 54 624 C1).

Die Kombination des neuen Stammes *Comamonas testosteroni* MC1 mit dem Stamm *Corynebacterium* sp. K2-17 ermöglicht überraschend den vollständigen Abbau sowohl in leicht sauren und neutralen pH-Bereichen als auch in alkalischem Milieu, wodurch z.B. das betroffene Mauerwerk oder entsprechende wäßrige Medien dekontaminiert werden.

Die Kultivierung der Stämme wird entweder getrennt oder im Gemisch durchgeführt. In einer bevorzugten Ausführungsvariante erfolgt eine direkte Kultivierung auf 2,4-DP, MCPP, 2,4-D, MCPA, DCP und/oder MCP sowie gegebenenfalls 2,4-DB und/oder MCPB enthaltenden Materialien, wodurch die Verbindungen in Gegenwart an sich üblicher Wachstumskomponenten vollständig abgebaut werden.

Das Verfahren wird vorteilhaft insbesondere zur Sanierung von Gebäudeabbruch aus Anlagen zur Produktion solcher Verbindungen bzw. mit solchen Substanzen kontaminierten Arealen bzw. Lager- und Umschlagplätzen, zur Behandlung von Wässern aus der Produktion solcher Verbindungen oder zur Behandlung von Böden, Wässern und Grundwässern im Umfeld der Produktion und Handhabung solcher Substanzen angewendet.

Die Erfindung wird an Ausführungsbeispielen näher erläutert:

### Beispiel 1

Der Stamm *C. testosteroni* MC1 wird auf einem Mineralmedium angezogen. Dieses Medium hat pro Gramm zu erzeugender Biomasse folgende Zusammensetzung (in mg/l) : NH₄Cl, 700; KH₂PO₄ 158; MgSO₄ * 7 H₂O, 8; CaCl₂ * 2 H₂O, 10; FeSO₄ * 7 H₂O, 2,5; ZnSO₄ * 7 H₂O, 0,23; MnSO₄ * 4 H₂O, 0,42; CuSO₄ * 5 H₂O, 0,39; Na₂MoO4, 0,12. Die Kultivierung erfolgt aerob bei einem pH-Wert von 8,5 bei 30° C. Als Kohlenstoff- und Energiequelle diente 2,4-DP in einer Konzentration von 2 g/l. Die Verdünnungsrate betrug 0,08 h⁻¹. Die Produktivität lag bei 0,02 g Biomassetrockensubstanz/l*h. Die so erzeugte Biomasse wurde belastetem Wasser, pH 9 zugesetzt, welches enthielt (mg/l): 2,4-D (6,5), MCPA (10,75), 2,4-DP (11,8), MCPP (4, 9) ; DCP (5,1), MCP (7,15) sowie 2,4-DB (2,75), MCPB ( 3,9). Zugabe *von C. testosteroni* MC1 in einer Konzentration von 0,12 g/l führte zu einem Abbau der Phenoxypropionat-Derivate und der substituierten Phenole innerhalb von 1 Stunde und der Phenoxyacetat-Derivate innerhalb von 3 Stunden.

### Beispiel 2

*C. testosteroni* MC1 wurde gemäß Beispiel 1 kultiviert. Als Kohlenstoff- und Energiequelle diente eine Mischung aus 2 g/l 2,4-DP und 2 g/l Na-Acetat. Die Verdünnungsrate betrug 0,12 h⁻¹. Die Produktivität lag bei 0,06 g Biomassetrockensubstanz /l*h. Die so erzeugte Biomasse wurde belastetem Wasser entsprechend der in Beispiel 1 aufgeführten Zusammensetzung zugesetzt. Der Abbau vollzog sich in ähnlichen Raten wie in Beispiel 1 gezeigt.

### Beispiel 3

*C. testosteroni* MC1 wurde gemäß Beispiel 1 kultiviert. Als Kohlenstoff- und Energiequelle diente eine Mischung aus 2 g/l 2,4-DP und 2 g/l Na-Acetat. Die Verdünnungsrate betrug 0,12 h⁻¹. Fünf Gramm (Biomassetrockensubstanz) der so erzeugten Biomasse wurden zur Inokulation eines Festbettreaktors eingesetzt. Dieser bestand aus Bauschuttgranula (hauptsächlich Beton) einer Korngröße von 5-10 mm. Das Volumen betrug 5 l. Nach Anheftung der Biomasse an die Matrix wurde ein herbizidbelasteter Flüssigkeitsstrom über den Festbettreaktor geleitet; der pH-Wert betrug 11,2, die Konzentration an 2,4-DP bzw. 2,4-D jeweils 80 mg/l. Belüftet wurde mit einer Rate von 2 m³/m³*h. Der Flüssigkeitsstrom wurde entsprechend der Verwertungskapazität eingestellt, so, daß die Konzentration im Auslauf des Reaktors gegen Null ging. Unter diesen Bedingungen konnte der Abbau sukzessive gesteigert und bei quantitativer Elimination eine stabile Abbauleistung von ca. 22 g Herbizid/m³*h erreicht werden. Die Phenoxybutyrat-Derivate wurden durch Inokulation mit dem Stamm *Corynebacterium* K2-17 (DE 196 54 624.9) quantitativ eliminiert.

### Beispiel 4

*C. testosteroni* MC1 wurde gemäß Beispiel 1 kultiviert. Als Kohlenstoff- und Energiequelle diente eine Mischung aus 5 g/l 2,4-DP und 5 g/l Na-Acetat. Die Verdünnungsrate betrug 0,12 h⁻¹. Dreißig Gramm (Biomassetrockensubstanz) der so erzeugten Biomasse wurden zur Inokulation eines Festbettreaktors eingesetzt. Dieser bestand aus Bauschuttgranula (hauptsächlich Beton) einer Korngröße von 5-10 mm. Das Volumen betrug 50 l. Belüftet wurde mit einer Rate von 2 m³/m³*h. Zugeführt wurde Eluat aus kontaminiertem Bauschutt, welches typischerweise enthielt (mg/l): 2,4-D (10), MCPA (20), 2,4-DP (20), MCPP (10); DCP (10), MCP (10) sowie 2,4-DB (10), MCPB (20). Der Abbau der Kontaminaten vollzog sich simultan. Bezüglich des Verwertungsspektrums für Stamm MC1 wurden Abbauleistungen von 18 g Herbizid(gemisch)/m³*h erreicht, die Effizienz des Abbaus lag bei diesen hohen Raten noch bei über 90 %. Die Phenoxybutyrat-Derivate wurden durch Inokulation mit dem Stamm *Corynebacterium* K2-17 (DE 196 54 624.9) quantitativ eliminiert.

## Patentansprüche

1. Bakterienstamm *Comamonas testosteroni* MC1 (DSM 12 392).

2. Verfahren zur mikrobiellen Dekontamination von Materialien und Wässern, die mit den folgenden Verbindungen der Phenoxyalkansäure-Herbizidproduktion 2, 4-Dichlorphenoxybuttersäure (2,4-DB), 4-Chlor-2-methylphenoxybuttersäure (MCPB), 2,4-Dichlorphenoxypropionsäure (2,4-DP), 4-Chlor-2-methylphenoxypropionsäure (MCPP), 2,4-Dichlorphenoxyessigsäure (2,4-D), 4-Chlor-2-methylphenoxyessigsäure (MCPA), 2,4-Dichlorphenol (DCP) und/oder 4-Chlor-2-methylphenol (MCP) bzw. mit deren Salzen belastet sind,
**dadurch gekennzeichnet, daß** man
zum Abbau von 2,4-DP, MCPP, 2,4-D, MCPA, DCP und/oder MCP den Bakterienstamm *Comamonas testosteroni* MC1 (DSM 12392) im leicht sauren bis alkalischen Bereich bei pH-Werten zwischen 6 und 11,5 unter aeroben Bedingungen bei Temperaturen zwischen 4 und 38 °C einsetzt.

3. Verfahren nach Anspruch 2,
**dadurch gekennzeichnet, daß** man
den Bakterienstamm *Comamonas testosteroni* MC1 in Kombination mit einem 2,4-DB/MCPB mineralisierenden Stamm einsetzt.

4. Verfahren nach Anspruch 3,
**dadurch gekennzeichnet, daß** man
als 2,4-DB und/oder MCPB mineralisierenden Stamm den Bakterienstamm *Corynebacterium sp. K2-17* (DSM 11288) einsetzt.

5. Verfahren nach einem der Ansprüche 2-4,
**dadurch gekennzeichnet, daß**
der Abbau der Verbindungen der Phenoxyalkansäure-Herbizidproduktion simultan erfolgt.

6. Verfahren nach einem der Ansprüche 2-5,
**dadurch gekennzeichnet, daß**
der Bakterienstamm *C. testosteroni* MC1 oder die Bakterienstämme *ex situ* getrennt oder im Gemisch kontinuierlich, diskontinuierlich oder mittels anderer Fütterungssstrategien nach an sich üblichen Methoden kultiviert und anschließend zur Dekontamination in Gegenwart an sich üblicher Wachstumskomponenten eingesetzt werden.

7. Verfahren nach einem der Ansprüche 2-5,
**dadurch gekennzeichnet, daß**
die Bakterienstämme direkt auf von 2,4-DP, MCPP, 2,4-D, MCPA, DCP und/oder MCP und auf gegebenenfalls 2,4-DB und/oder MCPB enthaltenden Materialien kontinuierlich, diskontinuierlich oder mittels anderer Fütterungssstrategien nach an sich üblichen Methoden kultiviert werden, wodurch die Verbindungen in Gegenwart an sich üblicher Wachstumskomponenten abgebaut werden.

8. Verfahren nach einem der Ansprüche 2-7,
**dadurch gekennzeichnet, daß**
das Dekontaminationsverfahren von den mit 2,4-DP, MCPP, 2,4-D, MCPA, DCP und/oder MCP und gegebenenfalls mit 2,4-DB/MCPB kontaminierten Wässern bzw. Materialien kontinuierlich, partiell kontinuierlich oder diskontinuierlich erfolgt.

9. Verfahren nach Anspruch 8,
**dadurch gekennzeichnet, daß**
die Dekontamination in belasteten Wässern erfolgt.

10. Verfahren nach Anspruch 8,
**dadurch gekennzeichnet, daß**
die Dekontamination von belasteten Materialien in wäßrigen Eluaten oder in befeuchtetem Zustand erfolgt.

11. Verfahren nach Anspruch 6,
**dadurch gekennzeichnet, daß**
der Bakterienstamm *C. testosteroni* MC1 *ex situ* unter Zusatz weiterer nicht chlororganischer heterotropher Auxiliarsubstrate als Kohlenstoff- und Energiequelle vermehrt wird.

12. Verfahren nach Anspruch 11,
**dadurch gekennzeichnet, daß**
die Kultivierung kontinuierlich erfolgt, wobei die Herbizide und die nicht chlororganischen heterotrophen Substrate in variablem Mischungsverhältnis vorliegen, vorzugsweise 10:1 bis 1:10 in g/g.

13. Verfahren nach Anspruch 8,
**dadurch gekennzeichnet, daß**
die Dekontamination diskontinuierlich erfolgt, indem Biomasse der Mikroorganismenstämme belasteten Wässern oder Materialien in einer Konzentration von vorzugsweise 0,01 bis 1 g pro Liter bzw. kg zugesetzt wird.

14. Verfahren nach einem der Ansprüche 2-13,
**dadurch gekennzeichnet, daß**
die Dekontamination unter Nutzung eines Festbettreaktors oder eines Flüssigphasenreaktors erfolgt.

## Claims

1. The bacterial strain *Comamonas testosteroni* MC1 (DSM 12392).

2. A process for the microbial decontamination of materials and waters polluted with the following compounds from the production of phenoxyalkanoic acid herbicides, 2,4-dichlorophenoxybutyric acid (2,4-DB), 4-chloro-2-methylphenoxybutyric acid (MCPB), 2,4-dichlorophenoxy-propionic acid (2,4-DP), 4-chloro-2-methylphenoxypropionic acid (MCPP), 2,4-dichlorophenoxyacetic acid (2,4-D), 4-chloro-2-methylphenoxyacetic acid (MCPA), 2,4-dichlorophenol (DCP), and/or 4-chloro-2-methylphenol (MCP), or salts thereof,
**characterized in that**
the bacterial strain *Comamonas testosteroni* MC1 (DSM 12392) in a range of from slightly acidic to alkaline at pH values between 6 and 11.5 under aerobic conditions at temperatures between 4 and 38°C is employed to degrade 2,4-DP, MCPP, 2,4-D, MCPA, DCP and/or MCP.

3. The process according to claim 2,
**characterized in that**
the bacterial strain *Comamonas testosteroni* MC1 is used in combination with a 2,4-DB/MCPB-mineralizing strain.

4. The process according to claim 3,
**characterized in that**
the bacterial strain *Corynebacterium sp. K2-17* (DSM 11288) is used as 2,4-DB- and/or MCPB-mineralizing strain.

5. The process according to any of claims 2-4,
**characterized in that**
the degradation of compounds from the production of phenoxyalkanoic acid herbicides is effected simultaneously.

6. The process according to any of claims 2-5,
**characterized in that**
the bacterial strain *C. testosteroni* MC1 or the bacterial strains are cultivated separately *ex situ* or in admixture in a continuous or discontinuous fashion, or by means of other feeding strategies according to per se usual methods, and subsequently employed in the decontamination in the presence of *per se* usual growth components.

7. The process according to any of claims 2-5,
**characterized in that**
the bacterial strains are cultivated directly on materials containing 2,4-DP, MCPP, 2,4-D, MCPA, DCP and/or MCP and on materials possibly containing 2,4-DB and/or MCPB in a continuous or discontinuous fashion or by means of other feeding strategies according to *per se* usual methods, so that these compounds will be degraded in the presence of *per se* usual growth components.

8. The process according to any of claims 2-7,
**characterized in that**
the decontamination process of waters or materials contaminated with 2,4-DP, MCPP, 2,4-D, MCPA, DCP and/or MCP and possibly with 2,4-DB/MCPB is conducted in a continuous, partially continuous or discontinuous fashion.

9. The process according to claim 8,
**characterized in that**
decontamination is effected in polluted waters.

10. The process according to claim 8,
**characterized in that**
decontamination of polluted materials is effected in aqueous eluates or in wetted condition.

11. The process according to claim 6,
**characterized in that**
the bacterial strain *C. testosteroni* MC1 is grown *ex situ* with addition of further non-chloroorganic heterotrophic auxiliary substrates as a source of carbon and energy.

12. The process according to claim 11,
**characterized in that**
culturing is effected continuously, the herbicides and non-chloroorganic heterotrophic substrates being present at varying mixing ratios, preferably from 10:1 to 1:10 in g/g.

13. The process according to claim 8,
**characterized in that**
decontamination is effected discontinuously by adding biomass of the strain of microorganisms to polluted waters or materials at a concentration of preferably 0.01 to 1 g per liter or kg.

14. The process according to any of claims 2-13,
**characterized in that**
decontamination is performed using a fixed-bed reactor or a liquid-phase reactor.

## Revendications

1. Souche bactérienne *Comamonas testosteroni* MC1 (DSM 12392).

2. Procédé de décontamination microbienne des matériaux et des eaux pollués par les composés suivants issus de la fabrication d'herbicides à l'acides phénoxyalcanoçques,
- acide 2,4-dichlorophénoxybutyrique (2,4-DB),
- acide 4-chloro-2-méthylphénoxybutyrique (MCPB),
- acide 2,4-dichlorophénoxyproprionique (2,4-DP),
- acide 4-chloro-2-méthylphénoxyproprionique (MCPP),
- acide 2,4-dichlorophénoxyacétique (2,4-D),
- acide 4-chloro-2-méthylphénoxyacétique (MCPA),
- 2,4-dichlorophénol (DCP) et/ou 4-chloro-2-méthylphénol (MCP),
ou leurs sels,
**caractérisé en ce que**,
pour la décomposition du 2,4-DP, du MCPP, du 2,4-D, du MCPA, du DCP et/ou du MCP, on utilise la souche bactérienne *Comamonas testosteroni* MC1 (DSM 12392) en milieu de légèrement acide à alcalin, à un pH compris entre 6 et 11,5, dans des conditions d'aérobie, à des températures comprises entre 4 et 38° C.

3. Procédé selon la revendication 2,
**caractérisé en ce**
**qu'**on utilise la souche bactérienne *Comamonas testosteroni* MC1 en association avec une souche minéralisant le 2,4-DB/MCPB.

4. Procédé selon la revendication 3,
**caractérisé en ce**
**qu'**on utilise, en tant que souche minéralisant le 2,4-DB et/ou le MCPB, la souche bactérienne *Corynebacterium sp. K2-17* (DSM 11288).

5. Procédé selon une des revendications de 2 à 4,
**caractérisé en ce que**
la dégradation des composés issus de la fabrication d'herbicides à l'acides phénoxyalcanoçques s'effectue de manière simultanée.

6. Procédé selon une des revendications de 2 à 5,
**caractérisé en ce**
**qu'**on cultive la souche bactérienne *C. testosteroni* MC1 ou les souches bactériennes de manière séparée *ex situ* ou mélangées, de manière continue, discontinue ou au moyen d'autres stratégies de nutrition, selon des méthodes habituelles en soi, et qu'ensuite on la/les utilise pour la décontamination en présence de milieux de croissance habituels en soi pour ces souches.

7. Procédé selon une des revendications de 2 à 5,
**caractérisé en ce**
**qu'**on cultive les souches bactériennes directement sur les matériaux contenant du 2,4-DP, du MCPP, du 2,4-D, du MCPA, du DCP et/ou du MCP, et éventuellement du 2,4-DB et/ou du MCPB de manière continue, discontinue ou par d'autres stratégies de nutrition, selon des méthodes habituelles en soi, par lesquelles les composés sont dégradés en présence de milieux de croissance habituels en soi pour ces souches.

8. Procédé selon une des revendications de 2 à 7,
**caractérisé en ce que**
le procédé de décontamination des eaux ou des matériaux contaminés par du 2,4-DP, du MCPP, du 2,4-D, du MCPA, du DCP et/ou du MCP, et éventuellement par du 2,4-DB/MCPB s'effectue de manière continue, de manière en partie continue ou de manière discontinue.

9. Procédé selon la revendication 8,
**caractérisé en ce que**
la décontamination s'effectue dans des eaux polluées.

10. Procédé selon la revendication 8,
**caractérisé en ce que**
la décontamination des matériaux pollués s'effectue dans des éluats aqueux ou à l'état humide.

11. Procédé selon la revendication 6,
**caractérisé en ce que**
la souche bactérienne *C. testosteroni* MC1 se multiplie ex *situ* en ajoutant, à titre de source d'énergie et de carbone, des substrats auxiliaires supplémentaires hétérotrophes ne contenant pas d'organochlorés.

12. Procédé selon la revendication 11,
**caractérisé en ce que**
la culture s'effectue de manière continue, avec un rapport de mélange variable entre les herbicides et les substrats hétérotrophes non organochlorés, qui est de préférence de 10 : 1 à 1 : 10 en g/g.

13. Procédé selon la revendication 8,
**caractérisé en ce que**
la décontamination s'effectue de manière discontinue, en ajoutant aux eaux ou aux matériaux pollués la biomasse des souches de microorganismes à une concentration de préférence de 0,01 à 1 g respectivement par litre ou par kg.

14. Procédé selon une des revendications de 2 à 13,
**caractérisé en ce que**
la décontamination s'effectue en utilisant un réacteur à lit fixe ou un réacteur à phase liquide.
